# EUROPEAN PATENT SPECIFICATION

(11) **EP 4 201 401 B1**
(45) Date of publication and mention of the grant of the patent: **11.02.2026**
(21) Application number: 22383251.0
(22) Date of filing: 21.12.2022
(51) Int. Cl.: A61K 31/00, A61K 9/107, A61P 1/00

(54) **OPHTHALMIC MICROEMULSION, PROCEDURE FOR OBTAINING AND USE THEREOF**
OPHTHALMISCHE MIKROEMULSION, VERFAHREN ZUR HERSTELLUNG UND VERWENDUNG DAVON
MICROEMULSION OPHTALMIQUE, PROCEDE D'OBTENTION ET UTILISATION

(30) Priority: 23.12.2021 ES 202131205
(43) Date of publication of application: 28.06.2023
(73) Proprietor: Universidad Complutense De Madrid, 28015 Madrid (ES)
(72) Inventor: LOPEZ CANO, Jose Javier, 28015 MADRID (ES); GONZALEZ CELA CASAMAYOR, Miriam Ana, 28015 MADRID (ES); BENITEZ DEL CASTILLO, Jose Manuel, 28015 MADRID (ES); HERRERO VANRELL, Rocio, 28015 MADRID (ES); MOLINA MARTÍNEZ, Irene Teresa, 28015 MADRID (ES); VICARIO DE LA TORRE, Marta, 28015 Madrid (ES)
(74) Representative: Herrero & Asociados, S.L.

(56) References cited:
- EP-A1- 3 919 047
- US-A1- 2012 251 613
- US-A1- 2020 390 699
- VANDAMME TH F: "Microemulsions as ocular drug delivery systems: recent developments and future challenges", PROGRESS IN RETINAL AND EYE RESEARCH, OXFORD, GB, vol. 21, no. 1, 1 January 2002 (2002-01-01), pages 15 - 34, XP002630484, ISSN: 1350-9462
- BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631 - 662, XP009102859, ISSN: 0193-2691, DOI: 10.1081/DIS-120015368

## Description

The present invention relates to ophthalmic microemulsions compatible with the precorneal film which can be used as artificial tears and as a drug vehicle for topical ocular administration. Likewise, it refers to the procedure for obtaining the microemulsion.

### Background of the invention

The ocular surface is composed of several layers among which are the corneal epithelium, conjunctival epithelium, meibomian glands and lacrimal glands.

A thin film known as precorneal or lacrimal film, with aqueous, mucinous and lipid components whose main function is to lubricate and protect the ocular surface, is distributed over these layers. This helps to prevent evaporation of the aqueous fraction and to preserve the normal osmolarity of the tear (≈300 mOsm/L). When there is excessive evaporation of the aqueous component or a defect in the secretion of the lipid component responsible for preventing evaporation of the aqueous fraction, there is an increase in tear osmolarity (>330 mOsm/L), which triggers a series of inflammatory processes associated with alterations in the ocular surface and loss of conjunctival and corneal epithelium cells, manifesting dry eye disease (EOS). These processes trigger a cascade of successive inflammatory events and cell death leading to a chronic disease.

Different topical ocular drug delivery systems have been developed with the aim of reducing dry eye symptoms and counteracting the various events of cell death and inflammation on the ocular surface. Liposomal formulations for the treatment of dry eye have been under research and development due to their ability to supply the precorneal film of the lipid film, as well as to encapsulate both hydrophilic and hydrophobic active substances.

Some authors have pointed out the potential of using nanoemulsions for the inclusion of hydrophilic and lipophilic active ingredients in the treatment of various ocular pathologies. Nanoemulsions are colloidal dispersions, both aqueous and oily, in which the dispersed phase forms nanometric droplets between 100 and 400 nm in the dispersing phase. These can be of the W/O or O/W type depending on which is the dispersing or majority phase (oil or water, respectively). In turn, they are characterized by the fact that they require an energy input for their production and are therefore thermodynamically unstable. Because of this, many nanoemulsions are produced by high-pressure homogenization, since it is necessary to overcome the barrier energy to form a stable system. Therefore, the development of emulsions that are easier to obtain is of great interest.

Along the same lines, an association between chronic use of antiglaucomatous agents and the onset, worsening or development of dry eye disease has been widely described. In many cases, antiglaucomatous formulations contain benzalkonium chloride as a preservative, which has toxicity on the corneal epithelium, conjunctival epithelium and the ocular surface in general. Benzalkonium chloride causes an increase in the expression of inflammatory markers in the corneal and conjunctival epithelium and decreases the density of goblet cells. These events lead to chronic inflammation, as well as destabilization of the prelagrimal film with the consequent appearance of a hyperosmolar environment. In many cases dry eye disease produced by prelagrimal film instability may be exacerbated by the administration of antiglaucomatous agents in a multiple-dose regimen. Therefore, the development of an antiglaucomatous product that does not involve all of these problems is of interest.

Patent EP1985298 describes an ocular emulsion containing prostaglandin.

The patent application US2020/390699 describes an oil-in-water microemulsion for formulating pharmaceutically active compounds. The microemulsion contain oil, surfactant, water, and the pharmaceutical compound. The patent application EP3919047 describes a microemulsion for ophthalmic applications such as treatment of dry eye syndrome, which comprises at least one oily component, an aqueous phase, at least one non-ionic surfactant, at least one polysaccharide, a salt or a derivative thereof, and at least one cross-linking agent. The document US2012/251613 discloses an ophthalmic bimatoprost microemulsion is described, where a microemulsion is a fluid composition containing an oil phase finely dispersed within a water phase. Finally, the document "Microemulsions as ocular drug delivery systems: recent developments and future challenges", Vandamme, Progress in retinal and eye research, Oxford, vol. 21, n°1, 1 January 2002 discloses microemulsions as ocular drug delivery systems. These documents do not describe an ophthalmic microemulsion wherein the oil phase comprises squalene.

The present invention describes an ocular microemulsion capable of being used as an artificial tear and as a vehicle for ocular medicaments.

### Description of the invention

In the present invention an ophthalmic microemulsion has been developed which have the ability to solubilize hydrophobic drugs that are difficult to vehicle in aqueous media and/or to increase the low bioavailability of those with decreased permeability.

Microemulsions are characterized by nanometer-sized droplet sizes, typically between 10 nm and 100 nm. The reduction of the droplet diameter implies an increase of its active surface, thus increasing the functionality of the medicinal substance and the therapeutic effect. In addition, they do not require energy input for their elaboration, being spontaneously and thermodynamically stable, with characteristics analogous to an ideal solution. Because of this, their stability profile can last for years, since their energy profile is very different from macro- and nanoemulsions. Like the previous ones, they can exist in various types depending on the nature of the dispersed and dispersing phase (w/o or o/w). Contrary to popular belief, the droplet size of nanoemulsions is between macroemulsions and microemulsions (approximately 100-500 nm).

Also, the nature of microemulsions is particularly attractive for the ocular route of administration because they form transparent solutions with low surface tension and very small droplet sizes, which promotes increased internalization of the active substances. The ophthalmic microemulsions of the invention are compatible with the precorneal film.

Therefore, microemulsions are presented as thermodynamically stable systems with a superior internalization capacity with potential use for transporting active substances into the cellular interior, specifically when it comes to barriers whose primary function is to prevent the passage of substances such as those present on the ocular surface.

The microemulsions include an aqueous and an oily phase and incorporate surfactants and cosurfactants for the formation of the microemulsion.

The microemulsions of the invention do not contain benzalkonium chloride.

Therefore, the first aspect of the invention relates to an ophthalmic microemulsion with a droplet size between 10 nm to 100 nm determined using the dynamic light scattering (DLS) method comprising:
an aqueous phase and
an oily phase comprising: a vegetable oil, a pharmaceutically acceptable fatty acid ester, a glycol compound, squalene, surfactants, and cosurfactants.

Preferably the droplet size is between 10 nm to 50 nm.

The term "glycol compound" refers to a compound with two or more hydroxyl substituents.

The term "vegetable oil" refers to a mixture of triglycerides of vegetable or similar origin. Examples of vegetable oils are argan oil, corn oil, palm oil, coconut oil, olive oil, peanut oil, rapeseed oil, sunflower oil, sesame oil, soybean oil.

The term "pharmaceutically acceptable fatty acid ester" refers to a C1-6 alkyl ester of a C10-20 fatty acid, which is suitable for use in contact with human and animal tissues without toxicity, irritation, allergic response.

The term "cosurfactants" refers to compounds which together with surfactants stabilize the microemulsion.

The process object of the invention described herein relates to the preparation of microemulsions in aqueous medium compatible with the precorneal film.

A second aspect of the invention relates to a procedure for obtaining the microemulsion of the invention comprising the steps of:
(a) mixing the compounds comprising the oily phase.
(b) adding the aqueous phase.
(c) filtering.
(d) stabilizing.

The latter aspects of the invention refer to uses which are the utilization of the microemulsion of the invention as artificial tears and as a vehicle of active principles with low bioavailability and/or poorly water-soluble to facilitate their administration by ocular topical route.

Thus, another aspect relates to the microemulsion for use as artificial tears. Likewise, the invention refers to the use of the microemulsion defined above for use as a drug vehicle for administration by the topical ocular route and for use in the treatment of ocular hypertension.

Finally, it also referred to the use of the microemulsion for use in the treatment of glaucoma.

### Brief description of the drawings

Figure 1 shows the morphology of microemulsion-base (MB) visualized by cryo-TEM at 73,000x magnification (a) and SEM at 200,000x magnification (b). The scale at the lower right comprises a size of 50 nm (a) and 200 nm (b). Microemulsions within the range described above are shown with an arrow.
Figure 2 shows a distribution of microemulsion-base (MB) sizes plotted on a semi-logarithmic scale expressed as % Chan (volume percent in the channel) versus size expressed in nanometers.
Figure 3 shows a rheology diagram obtained for the microemulsion-base (MB) viscosity study. Shear stress (Pa) and viscosity () versus shear rate (1/s) are plotted.
Figure 4 shows cell viability (%) with the base vehicle incubated at 37°C for 1 and 4 hours in corneal and conjunctival epithelial cell lines. Two controls are used, one positive (+; benzalkonium chloride 0.005%) and one negative (-; NaCl 0.9% aqueous solution).
Figure 5 shows results of the cellular uptake assay of the microemulsion-base (MB) formulation by human corneal epithelial cells (HCE). Shown are HCE cells exposed to a 10 µg/mL coumarin suspension for 15 minutes (a), MB loaded with 10 µg/mL coumarin for 15 minutes (b), 10 µg/mL coumarin suspension for 30 minutes (c) and to MB loaded with 10 µg/mL coumarin for 30 minutes (d). The scale bar represents 50 µm.
Figure 6 shows a morphology of the microemulsion-base with hyaluronic acid (MB-HA) visualized by cryo-TEM at 73,000x magnification (a) and SEM at 200,000x magnification (b). The scale at the lower right comprises a size of 50 nm (a) and 200 nm (b). Microemulsions within the range described above are shown with an arrow.
Figure 7 shows a size distribution of the microemulsion-base with hyaluronic acid (MB-HA) represented in semi-logarithmic scale expressed as % Chan (volume percentage in the channel) versus size expressed in nanometers.
Figure 8 shows a rheology diagram obtained for the viscosity study of the microemulsion-base with hyaluronic acid (MB-HA). Shear stress (Pa) and viscosity () are plotted versus shear rate (1/s).
Figure 9 shows the cell viability (%) of the microemulsion with sodium hyaluronate (MB-HNa) incubated at 37°C for 1 and 4 hours in corneal and conjunctival epithelial cell lines. Two controls are used, one positive (+; benzalkonium chloride 0.005%) and one negative (-; NaCl 0.9% aqueous solution).
Figure 10 shows the cell viability (%) of microemulsion with sodium hyaluronate with osmoprotective and re-epithelializing substances incubated at 37°C for 1 and 4 hours in corneal epithelial cell lines. Two controls are used, one positive (+; benzalkonium chloride 0.005%) and one negative (-; NaCl 0.9% aqueous solution).
Figure 11 shows intraocular pressures (expressed as a percentage of baseline pressure) obtained with the MB-HA formulation (ΔPIO>10 %) compared to those found with the microemulsion-base used as a control (ΔPIO<10 %).
Figure 12 shows a morphology of the microemulsion with sodium hyaluronate and latanoprost (MB-HNa-Latanoprost) visualized by cryo-TEM at 73000 magnification (a) and SEM at 100000 magnifications (b). The scale at the lower right comprises a size of 50 nm (a) and 200 nm (b). Microemulsions comprised in the range described above are shown with an arrow.
Figure 13 shows a size distribution of the microemulsion with hyaluronic acid and latanoprost (MB-HA-Latanoprost) represented in semi-logarithmic scale expressed as % Chan (volume percentage in the channel) versus size expressed in nanometers.
Figure 14 shows a rheology diagram obtained for the viscosity study of the microemulsion with hyaluronic acid and latanoprost (MB-HA-Latanoprost). Shear stress (Pa) and viscosity (Pa-s) versus shear rate (1/s) are plotted.
Figure 15 shows the cell viability (%) of microemulsion with sodium hyaluronate and latanoprost (MB-HNa-Latanoprost) incubated at 37°C for 1 and 4 hours in corneal and conjunctival epithelial cell lines. Two controls are used, one positive (+; benzalkonium chloride 0.005%) and one negative (-; NaCl 0.9% aqueous solution).
Figure 16 shows intraocular pressures expressed as a percentage of baseline pressure obtained with the MB-HA-Latanoprost formulation and the commercial Monoprost used as a reference in the assay. Microemulsion-base (MB) is used as the control formulation.

### Description of a preferred embodiment

As stated the first aspect of the invention relates to an ophthalmic microemulsion with a droplet size between 10 nm to 100 nm determined using the dynamic light scattering (DLS) method comprising:
an aqueous phase and
an oily phase comprising: a vegetable oil, a pharmaceutically acceptable fatty acid ester, a glycol compound, squalene, a surfactant and a cosurfactant.

Preferably the vegetable oil is at a mass concentration with respect to the total mass of the microemulsion in a range between 0.001% and 2%, the pharmaceutically acceptable fatty acid ester at a mass concentration with respect to the total mass of the microemulsion in a range between 0.05% and 4% and the glycol compound is at a mass concentration with respect to the total mass of the microemulsion in a range between 0.5% and 4%.

In particular manner in the oily phase of the invention the vegetable oil is soybean oil, the pharmaceutically acceptable fatty acid ester is ethyl oleate and the glycol compound is propylene glycol which favors the dissolution of the hydrophobic components.

Preferably the surfactant of the microemulsion is at a mass concentration with respect to the total mass of the microemulsion in a range between 0.1% and 2% and the cosurfactant is at a mass concentration with respect to the total mass of the microemulsion in a range between 0.5% and 3%.

In particular as surfactants of the microemulsion is soy phosphatidylcholine and the co-surfactant is polyoxyl ricin 35 (Kolliphor EL^{®}). Specifically, phosphatidylcholine, after administration, will decrease the surface tension of the tear film, which favors its rapid extensibility and interaction with the mucins of the ocular surface.

In general, different active compounds can be added to microemulsions which, without being drugs, have osmoprotective, re-epithelializing, anti-inflammatory and/or antioxidant properties and protect the ocular surface from possible adverse effects that may occur with the continued use of the preparation for long periods of time. These compounds according to their nature will be included in the aqueous phase or in the oily phase.

Preferably the oily phase comprises liposoluble actives selected among: oleanolic acid, ubiquinol and/or liposoluble vitamins.

Preferably the squalene is in a mass concentration with respect to the total mass of the microemulsion in a range between 0.1% and 2%.

Preferably the microemulsion comprises an antiglaucomatous medicinal substance, more preferably prostaglandins, in particular the oily phase comprises latanoprost, CAS nuBMer 130209-82-4. Latanoprost belongs to the group of drugs known as prostaglandin analogues. It is used to treat diseases such as open-angle glaucoma and ocular hypertension in adults. Both diseases are associated with increased pressure inside the eye, which can affect vision. Latanoprost works by increasing the natural outflow of aqueous humor from inside the eye into the bloodstream.

Preferably the aqueous phase comprises mucoadhesive and/or mucomimetic compounds. Preferably the mucoadhesive and/or mucomimetic compound of the microemulsion is in a mass concentration with respect to the total mass of the microemulsion in a proportion of less than 0.8%. More preferably the mucoadhesive and/or mucomimetic compounds are selected from: hyaluronic acid and its salts, dextran, cellulose derivatives, chondroitin sulfate, chitosan, alginates, and gelane gum.

In particular, the compound is hyaluronic acid or its salts, presenting characteristics similar to those of the precorneal film. This microemulsion can be used as artificial tears.

In the aqueous phase the isotonizing agent (trehalose, sodium chloride, glucose) is incorporated to achieve an adjusted osmolarity for clinical use and the appropriate agent to buffer the preparation to a suitable pH. The preparations may be isotonic or hypotonic.

Therefore, preferably the aqueous phase comprises an isotonizing agent and more preferably the isotonizing agent is selected from: trehalose, sodium chloride, glucose, fructose, mannitol, sorbitol, ribitol, erythritol and dextrose.

Preferably the aqueous phase comprises water-soluble actives selected among water-soluble vitamins, betaine, leucine, clusterin and L-carnitine, taurine, glycine, ribitol, ectoine and amino acids. In particular betaine, leucine.

Preferably the aqueous phase has an osmolarity comprised in a range between 150 mOsm/kg and 330 mOsm/kg.

Preferably the microemulsion comprises agents capable of lowering intraocular pressure, neuroprotective agents, genetic material such as DNA and RNA, proteins and nucleic acids, immunogens such as squalene and polysorbate 80.

Another aspect of the invention relates to a procedure for obtaining the microemulsion of the invention comprising the steps of:
(a) mixing the compounds comprising the oily phase.
(b) adding the aqueous phase.
(c) filtering.
(d) stabilizing.

Preferably stage a) is carried out at a temperature between 20°C and 30°C and with protection from light, in stage b) the aqueous phase is added to the oily phase under agitation, in stage c) filtering is carried out with a filter having a pore size between 0.20 µm and 0.25 µm and wherein stage d) of stabilization is carried out at a temperature between 2°C and 25°C.

### EXAMPLES

The following examples have only illustrative character of this invention and should not be interpreted in a limiting sense thereof.

### Example 1

### 1° Preparation of the phases

OLEOUS PHASE (for 10 g of microemulsion). Ethyl oleate 0.8 % (m:m), soybean oil 0.2 % (m:m), Kolliphor EL^{®} 1 % (m:m), propylene glycol 1 % (m:m), soybean phosphatidylcholine 0.5 % (m:m) and squalene 0.2 % (m:m) were used.

The different components were mixed and left in agitation at 400 rpm, and at room temperature, for a minimum of 30 min until transparency was achieved.

The osmolarity of the oily phase, without liposoluble actives, is close to 170 mOsm/L.

AQUEOUS PHASE (for 10 g of microemulsion). Hyaluronic acid 0.2 % (m:m), trehalose cs. for desired osmolarity (250-300 mOsm/kg), water csp. 10 g of microemulsion (first aqueous phase) were used.

A second aqueous phase equal to the previous one was prepared without hyaluronic acid. The microemulsion prepared with the aqueous phase without hyaluronic acid is hereafter referred to as MB, microemulsion base.

And a third aqueous phase with hyaluronic acid with the addition of osmoprotective and re-epithelializing active ingredients to this formulation which favors its use as a vehicle for ocular hypotensive drugs as prolonged treatments and which make possible the manifestation of ocular surface alterations.

### 2° Elaboration of the microemulsion

The microemulsion was prepared at room temperature and protected from light. The totality of any of the aqueous phases was added, in a single step, to the oily phase which was agitated at 1,000 rpm. The agitation was maintained at 1,000 rpm for 10 minutes. It was filtered, using a low retention hydrophilic filter with a pore size of 0.22 µm. It was left to stabilize in the refrigerator at 2-8°C for 24 hours.

### 3° Characteristics of the microemulsion obtained in points 1 and 2, referenced as MB (microemulsion base without hyaluronic acid).

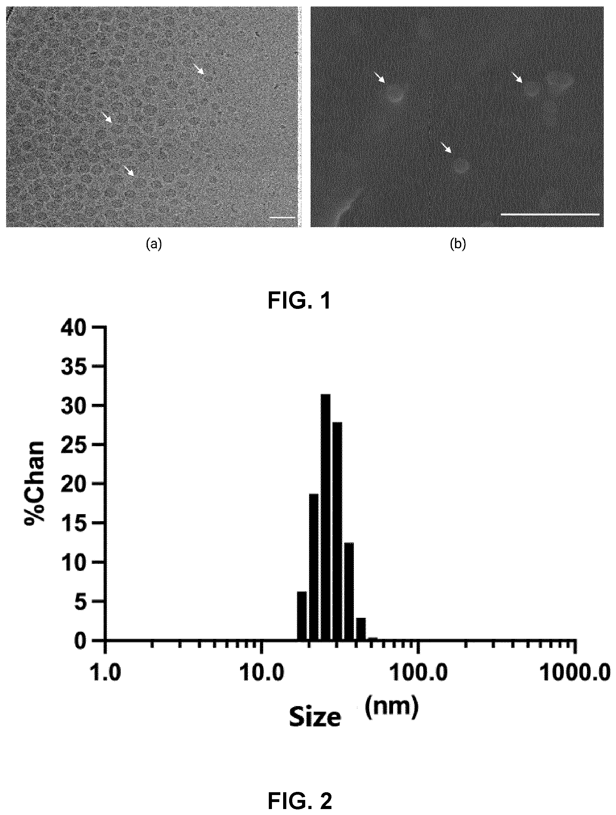

This characterization is limited to the microemulsion as described in this example. The morphology of the droplets was studied using cryo-TEM and SEM techniques. For this purpose, 200 kV FEI TALOS Arctica and JEOL JSM 7600F equipment were used, respectively. The photographs obtained are shown in Figure 1 (a) and (b).

The MB preparation presents pH values of 7.27 ± 0.12.

The diameter of the emulsified microemulsion particles was determined using the dynamic light scattering (DLS) method (also called photon correlation spectroscopy). The average volume diameter value of the particles obtained in different batches of the microemulsion ranged from 21 to 23 nm with standard deviations (SD) between 4.5 and 5.3 nm. The percentage of particles above 50 nm was, in all cases, less than 1 % (Figure 2).

Zeta potential values for the different batches ranged from -7 to -9 mV (measurements were performed at room temperature with an Autosizer 4700, Malvern).

Surface tension measurements were performed with a Kruss commercial brand tensiometer (K-11) using the plate method and values between 34 and 38 mN/m were obtained.

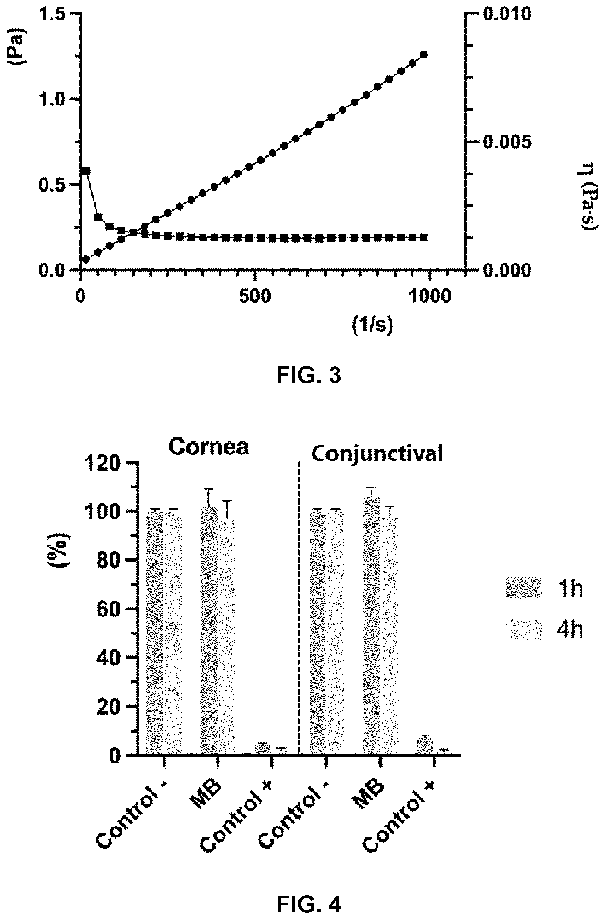

In the rheological evaluation of the MB vehicle at shear velocities between 0 and 1,000 s-1 and room temperature, it was observed that a Newtonian behavior was observed with dynamic viscosity values between 1.1 and 1.3 mPa-s (Figure 3). The Discovery HR-Hybrid Rheometer DHR-1 with a 60 mm parallel plate system was used for its determination.

Human corneal (hTERT-HCEC) and human conjunctival (HConEpiC) epithelial cell lines were used in the in vitro cytotoxicity assays. The mitochondrial reduction technique of 3-(4,5-dimethylthiazol-2-yl)-2,5-diphenyltetrazolium bromide salt (MTT) to a colored product (formazan) was used for the study (Mossman T. J Immun Methods 1983, 65:55-63). Sodium chloride 0.9% was used as a negative control and benzalkonium chloride 0.005% as a positive control. The solutions were incubated at 37 °C for 1 and 4 hours. The results obtained showed a tolerance of more than 90 % at the times tested, which is considered to be optimal (Figure 4).

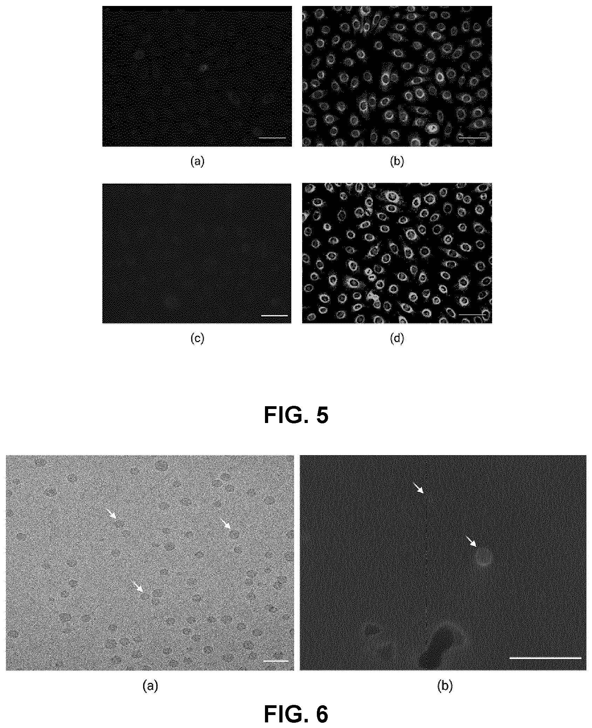

In an initial assay of cellular uptake of lipid droplets by human corneal epithelial cells (hTERT-HCEC) using the microemulsion labeled with a fluorescent dye, coumarin-6, a very lipophilic substance, uptake was manifested at 15 and 30 min of exposure as shown in Figure 5.

### 4° Characteristics of the microemulsion according to point 1 and 2 with 0.2% sodium hyaluronate as mucoadhesive polymer (called MB-HNa).

The characterization of this formulation (MB-HNa) was carried out using the same equipment as those indicated in section 3.

The morphology of the droplets was studied using cryo-TEM and SEM techniques. The photographs obtained are shown in Figures 6 (a) and (b).

The MB-HNa preparation shows pH values between 6.79 ± 0.05.

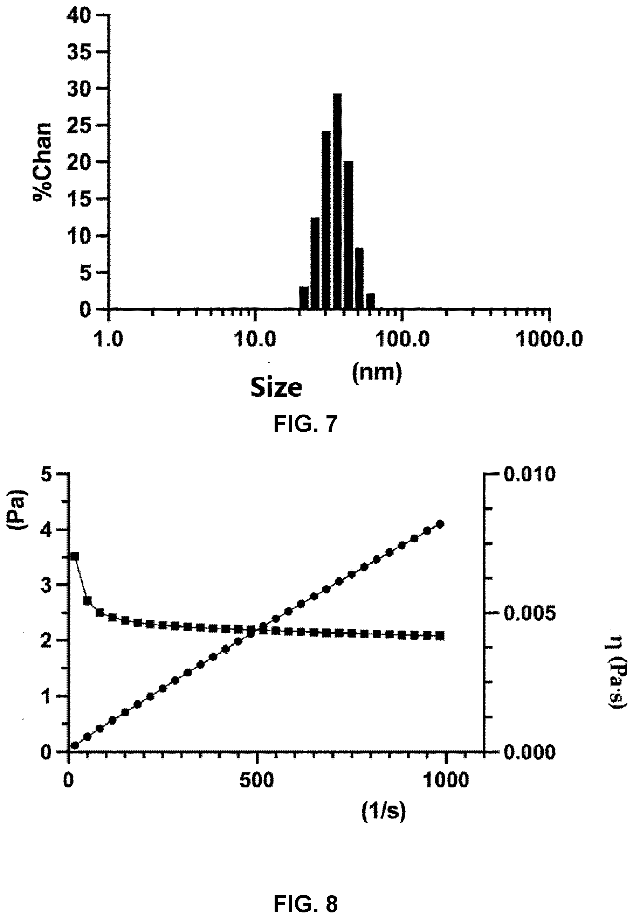

The volume mean diameter of the particles obtained in different batches of the microemulsion with sodium hyaluronate ranged between 29 and 37 nm with standard deviations (SD) between 6.9 and 7.4 nm. The percentage of particles larger than 50 nm was less than 11 % (Figure 7).

Zeta potential fluctuated between -0.1 and 0.2 mV (measurements were performed at room temperature).

Surface tension measurements on the vehicle of the invention with sodium hyaluronate found values between 35 and 38 mN/m.

In the rheological evaluation of the room temperature vehicle of the invention with sodium hyaluronate at shear rates between 0 and 1,000 s-1 a Newtonian behavior is manifested with dynamic viscosity values between 4.3 and 4.5 mPa-s (Figure 8).

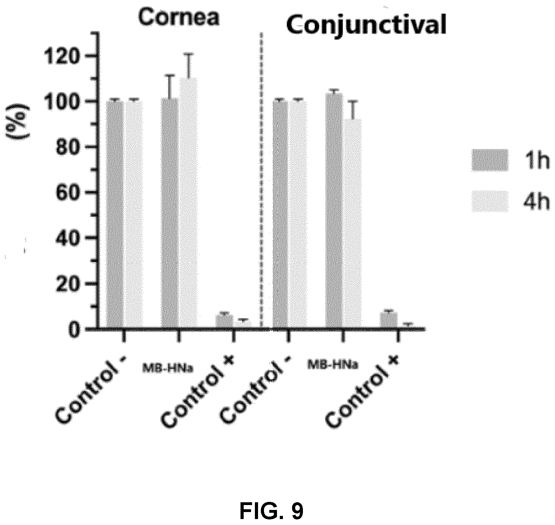

Human corneal (hTERT-HCEC) and human conjunctival (HConEpiC) epithelial cell lines were used in the in vitro cytotoxicity assays. The mitochondrial reduction technique of 3-(4,5-dimethylthiazol-2-yl)-2,5-diphenyltetrazolium bromide salt (MTT) to a colored product (formazan) was used for the study (Mossman T. J Immun Methods 1983, 65:55-63). Sodium chloride 0.9% was used as a negative control and benzalkonium chloride 0.005% was used as a positive control. The solutions were incubated at 37 °C for 1 and 4 hours. The results obtained showed a tolerance of more than 90 % at all the times tested (Figure 9).

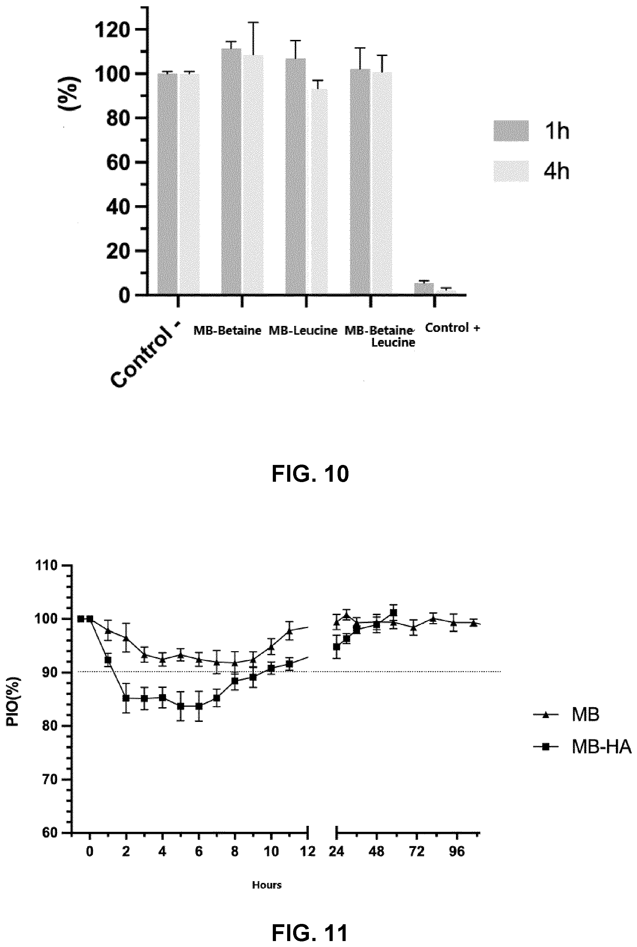

This pharmaceutical composition (MB-HNa) with preocular film characteristics has the ability to decrease ocular tension in the rabbit. This can be seen in the results obtained in this animal species after administration of 25 µL of the formulation (Figure 11). A decrease in intraocular pressure of 15 to 17% is observed 2 hours after instillation, which is maintained for 6 hours.

### 5° Characteristics of the microemulsion according to the invention with optional agents

To this vehicle (MB-HNa) based on the microemulsion object of the invention with sodium hyaluronate can be added optional osmoprotective and re-epithelializing components such as betaine, leucine and I-carnitine. There is no commercially available artificial tear that is a microemulsion including these components.

In the studies carried out with the human corneal cell line (hTERT-HCEC), formulations with optional osmoprotective and re-epithelializing substances such as betaine and leucine were included, maintaining good tolerance in the formulations that include the indicated active ingredients (Figure 10). This composition derived from the microemulsion object of the invention due to the characteristics indicated in this section can be used as a lacrimal substitute. This fact, together with the addition of osmoprotective and reepithelializing active ingredients to this formulation, favors its use as a vehicle for ocular hypotensive drugs that involve prolonged treatments and make possible the manifestation of ocular surface alterations.

### 6° Characteristics of the microemulsion according to the invention with optional agents and an antiglaucomatous medicinal substance.

The last test was performed with a microemulsion comprising the base composition described in the first point of this example, sodium hyaluronate and latanoprost.

The eye drops marketed as a solution in multidose containers contain benzalkonium chloride, a product that is highly cytotoxic to conjunctival and corneal cells. The product marketed in single-dose containers (Monoprost) incorporates different surfactants other than benzalkonium chloride. The latter eye drops have been used as a reference in hypotensive activity comparison tests.

The pharmaceutical preparation intended for this invention carries the same dosage as the preparations that are commercially available (50 µg/mL).

The characterization of this formulation (MB-HNa-Latanoprost) was carried out using the same equipment as those indicated in section 3.

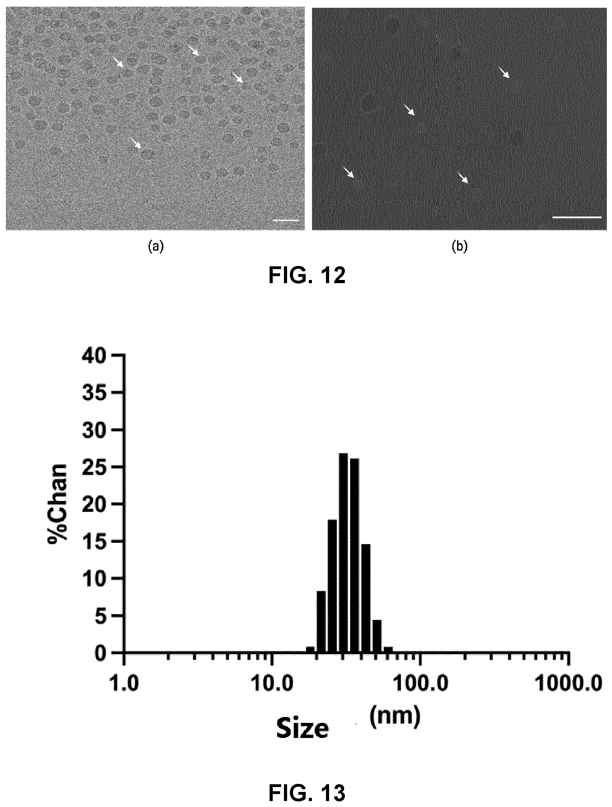

The morphology of the droplets was studied using cryo-TEM and SEM. The photographs obtained are shown in Figures 12 (a) and (b).

The MB-HNa-latanoprost preparation presents a pH value of 6.58 ± 0.02.

The average volume diameter of the particles obtained in different batches of the microemulsion with sodium hyaluronate ranged between 29 and 32 nm with standard deviations (SD) between 6.9 and 7.1 nm. The percentage of particles above 50 nm was less than 6 % (Figure 13).

Zeta potential ranged from -0.1 to 0.0 mV and surface tension values ranged from 36 to 38 mN/m.

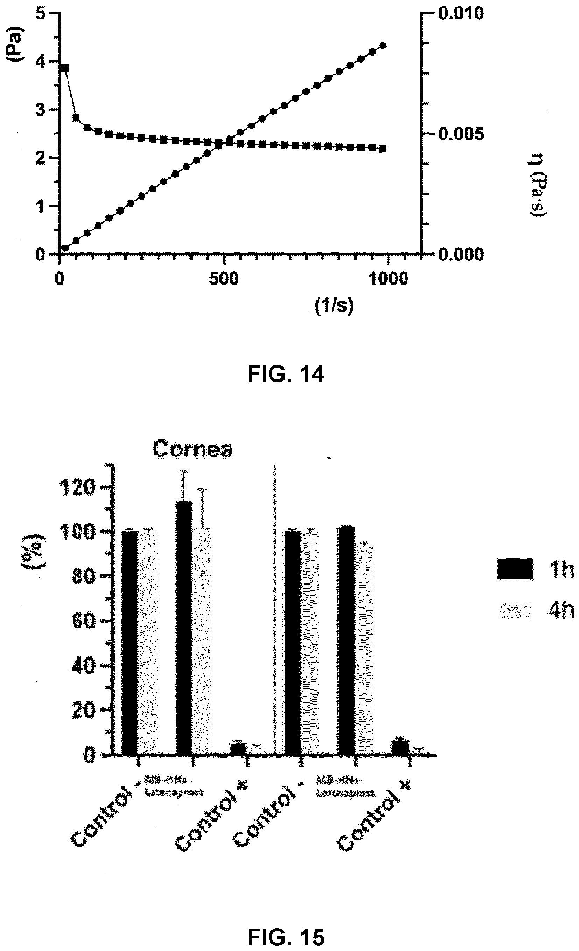

In the rheological evaluation of the microemulsion with sodium hyaluronate and latanoprost performed at shear rates between 0 and 1,000 s-1, a Newtonian behavior is manifested with dynamic viscosity values between 4.5 and 4.7 mPa-s (Figure 14).

Human corneal (hTERT-HCEC) and human conjunctival (HConEpiC) epithelial cell lines were used in the in vitro cytotoxicity assays. The mitochondrial reduction technique of 3-(4,5-dimethylthiazol-2-yl)-2,5-diphenyltetrazolium bromide salt (MTT) to a colored product (formazan) was used for the study (Mossman T. J Immun Methods 1983, 65:55-63). Sodium chloride 0.9% was used as a negative control and benzalkonium chloride 0.005% was used as a positive control. The solutions were incubated at 37°C for 1 and 4 hours. The results obtained showed a tolerance of more than 90 % at all the times tested (Figure 15).

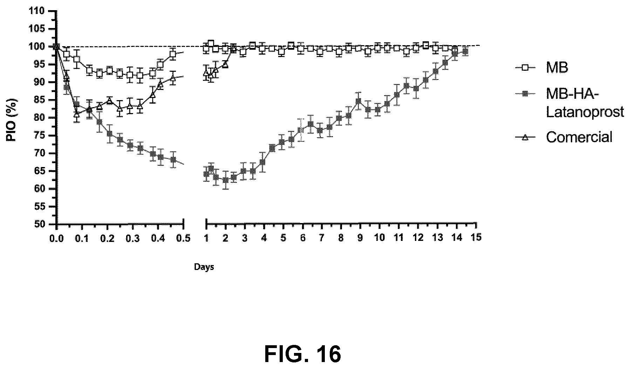

This pharmaceutical composition (MB-HNa-latanoprost) with hypotensive activity was compared with the commercially available formulation of latanoprost in single-dose containers (Monoprost) used as a reference. The base microemulsion (MB) was used as a control. The results are shown in Figure 16. At 2 h after administration, a decrease in intraocular pressure (ΔPIO) of more than 15% was achieved with both formulations. In the case of the commercial formulation, a maximum hypotensive effect of 20% was achieved at 2h which was preserved for approximately 9 h (ΔPIO ~15 %) and a residual effect (less than 10 %) was maintained up to 2 days. With the pharmaceutical composition that is the subject of the patent, in addition a ΔPIO between 35-38 % can be observed starting at 24 h which is maintained for 3 days. From the fourth day on, the effect begins to decrease slowly, maintaining the hypotensive efficacy (ΔPIO>10%) up to 12 days.

## Claims

1. An ophthalmic microemulsion with a droplet size between 10 nm to 100 nm determined using the dynamic light scattering (DLS) method comprising:
an aqueous phase and
an oil phase comprising: a vegetable oil, a pharmaceutically acceptable fatty acid ester, a glycol compound, squalene, surfactants and cosurfactants.

2. The microemulsion of claim 1 **characterized in that** the vegetable oil is soybean oil, the pharmaceutically acceptable fatty acid ester is ethyl oleate and the glycol compound is propylene glycol, the surfactant is soybean phosphatidylcholine and the co-surfactant is polyoxyl castor oil 35.

3. The microemulsion according to any one of claims 1 to 2 **characterized in that** the aqueous phase comprises mucoadhesive and/or mucomimetic compounds are selected from: hyaluronic acid and its salts, dextran, cellulose derivatives, chondroitin sulfate, chitosan, dextran, alginates and gelane gum.

4. The microemulsion according to any one of claims 1 to 3 **characterized in that** the oil phase comprises lipid-soluble actives selected from oleanolic acid, ubiquinol, and/or lipid-soluble vitamins.

5. The microemulsion according to any one of claims 1 to 4 **characterized in that** the oily phase comprises latanoprost.

6. The microemulsion according to any one of claims 1 to 5 **characterized in that** the aqueous phase comprises an isotonic agent is selected from trehalose, sodium chloride, glucose, fructose, mannitol, sorbitol, ribitol, erythritol, and dextrose.

7. The microemulsion according to any one of claims 1 to 6 **characterized in that** the aqueous phase comprises water-soluble actives selected from: water-soluble vitamins, betaine, clusterin, leucine, L-carnitine, taurine, glycine, ribitol, ectoine and amino acids.

8. The microemulsion according to any one of claims 1 to 7, **characterized in that** the aqueous phase has an osmolarity between 150 mOsm/kg and 330 mOsm/kg.

9. A process for obtaining the microemulsion according to claims 1 to 8 comprising the steps of:
(a) mixing the compounds comprising the oily phase.
(b) adding the aqueous phase.
(c) filtering.
(d) stabilizing.

10. The process according to claim 9 **characterized in that**:
step a) is carried out at a temperature between 20°C and 30°C and with protection from light, in step b) the aqueous phase is added in one step to the oily phase under agitation, in step c) the filtering is carried out with a filter having a pore size between 0.20 µm and 0.25 µm and wherein step d) of stabilization is carried out at a temperature between 2°C and 25°C.

11. The microemulsion according to claims 3 to 4 and 6 to 8 for use as artificial tears.

12. The microemulsion according to claims 1 to 4 and 6 to 8 for use as a drug vehicle for topical ocular administration.

13. The microemulsion according to any one of claims 5 to 8 for use in the treatment of ocular hypertension.

14. The microemulsion according to claim 5 to 8 for use in the treatment of glaucoma.

## Patentansprüche

1. Ophthalmische Mikroemulsion mit einer Tröpfchengröße zwischen 10 nm und 100 nm, bestimmt unter Verwendung des Verfahrens der dynamischen Lichtstreuung (DLS), umfassend:
eine wässrige Phase und
eine Ölphase, umfassend: ein Pflanzenöl, einen pharmazeutisch akzeptablen Fettsäureester, eine Glykolverbindung, Squalen, Tenside und Cotenside.

2. Mikroemulsion nach Anspruch 1, **dadurch gekennzeichnet, dass** das Pflanzenöl Sojabohnenöl ist, der pharmazeutisch akzeptable Fettsäureester Ethyloleat ist und die Glykolverbindung Propylenglykol ist, das Tensid Sojabohnenphosphatidylcholin ist und das Cotensid Polyoxylrizinusöl 35 ist.

3. Mikroemulsion nach einem der Ansprüche 1 bis 2, **dadurch gekennzeichnet, dass** die wässrige Phase mucoadhäsive und/oder mucomimetische Verbindungen umfasst, die ausgewählt sind aus: Hyaluronsäure und ihren Salzen, Dextran, Cellulosederivaten, Chondroitinsulfat, Chitosan, Dextran, Alginaten und Gelangummi.

4. Mikroemulsion nach einem der Ansprüche 1 bis 3, **dadurch gekennzeichnet, dass** die Ölphase lipidlösliche Wirkstoffe umfasst, die ausgewählt sind aus Oleanolsäure, Ubiquinol und/oder lipidlöslichen Vitaminen.

5. Mikroemulsion nach einem der Ansprüche 1 bis 4, **dadurch gekennzeichnet, dass** die ölige Phase Latanoprost umfasst.

6. Mikroemulsion nach einem der Ansprüche 1 bis 5, **dadurch gekennzeichnet, dass** die wässrige Phase ein isotonisches Mittel umfasst, das ausgewählt ist aus Trehalose, Natriumchlorid, Glucose, Fructose, Mannitol, Sorbitol, Ribitol, Erythritol und Dextrose.

7. Mikroemulsion nach einem der Ansprüche 1 bis 6, **dadurch gekennzeichnet, dass** die wässrige Phase wasserlösliche Wirkstoffe umfasst, die ausgewählt sind aus: wasserlöslichen Vitaminen, Betain, Clusterin, Leucin, L-Carnitin, Taurin, Glycin, Ribitol, Ectoin und Aminosäuren.

8. Mikroemulsion nach einem der Ansprüche 1 bis 7, **dadurch gekennzeichnet, dass** die wässrige Phase eine Osmolarität zwischen 150 mOsm/kg und 330 mOsm/kg aufweist.

9. Verfahren zum Erhalten der Mikroemulsion nach den Ansprüchen 1 bis 8, umfassend die Schritte:
(a) Mischen der Verbindungen, die die ölige Phase bilden.
(b) Zugeben der wässrigen Phase.
(c) Filtrieren.
(d) Stabilisieren.

10. Verfahren nach Anspruch 9, **dadurch gekennzeichnet, dass**: Schritt a) bei einer Temperatur zwischen 20°C und 30°C und unter Schutz vor Licht durchgeführt wird, in Schritt b) die wässrige Phase in einem Schritt zu der öligen Phase unter Rühren gegeben wird, in Schritt c) das Filtrieren mit einem Filter mit einer Porengröße zwischen 0,20 µm und 0,25 µm durchgeführt wird und wobei Schritt d) des Stabilisierens bei einer Temperatur zwischen 2°C und 25°C durchgeführt wird.

11. Mikroemulsion nach den Ansprüchen 3 bis 4 und 6 bis 8 zur Verwendung als künstliche Tränen.

12. Mikroemulsion nach den Ansprüchen 1 bis 4 und 6 bis 8 zur Verwendung als Arzneimittelvehikel zur topischen okularen Verabreichung.

13. Mikroemulsion nach einem der Ansprüche 5 bis 8 zur Verwendung bei der Behandlung von okularer Hypertonie.

14. Mikroemulsion nach Anspruch 5 bis 8 zur Verwendung bei der Behandlung von Glaukom.

## Revendications

1. Microémulsion ophtalmique d'une taille de gouttelette comprise entre 10 nm et 100 nm déterminée à l'aide de la méthode de diffusion dynamique de la lumière (DLS) comprenant :
une phase aqueuse et
une phase huileuse comprenant : une huile végétale, un ester d'acide gras pharmaceutiquement acceptable, un composé glycol, du squalène, des tensioactifs et des co-tensioactifs.

2. Microémulsion selon la revendication 1, **caractérisée en ce que** l'huile végétale est de l'huile de soja, l'ester d'acide gras pharmaceutiquement acceptable est de l'oléate d'éthyle et le composé glycol est du propylène glycol, le tensioactif est de la phosphatidylcholine de soja et le co-tensioactif est de l'huile de ricin polyoxylique 35.

3. Microémulsion selon l'une quelconque des revendications 1 et 2, **caractérisée en ce que** la phase aqueuse comprend des composés mucoadhésifs et/ou mucomimétiques choisis parmi : l'acide hyaluronique et ses sels, le dextran, les dérivés de cellulose, le sulfate de chondroïtine, le chitosan, les alginates et la gomme de gélane.

4. Microémulsion selon l'une quelconque des revendications 1 à 3, **caractérisée en ce que** la phase huileuse comprend des actifs liposolubles choisis parmi l'acide oléanolique, l'ubiquinol et/ou des vitamines liposolubles.

5. Microémulsion selon l'une quelconque des revendications 1 à 4 **caractérisée en ce que** la phase huileuse comprend du latanoprost.

6. Microémulsion selon l'une quelconque des revendications 1 à 5, **caractérisée en ce que** la phase aqueuse comprend un agent isotonique choisi parmi le tréhalose, le chlorure de sodium, le glucose, le fructose, le mannitol, le sorbitol, le ribitol, l'érythritol et le dextrose.

7. Microémulsion selon l'une quelconque des revendications 1 à 6, **caractérisée en ce que** la phase aqueuse comprend des actifs solubles dans l'eau choisis parmi : les vitamines solubles dans l'eau, la bétaïne, la clusterine, la leucine, la L-carnitine, la taurine, la glycine, le ribitol, l'ectoïne et les acides aminés.

8. Microémulsion selon l'une quelconque des revendications 1 à 7, **caractérisée en ce que** la phase aqueuse présente une osmolarité comprise entre 150 mOsm/kg et 330 mOsm/kg.

9. Procédé pour obtenir la microémulsion selon les revendications 1 à 8, comprenant les étapes suivantes :
(a) le mélange des composés comprenant la phase huileuse ;
(b) l'ajout de la phase aqueuse ;
(c) le filtrage ;
(d) la stabilisation.

10. Procédé selon la revendication 9, **caractérisé en ce que** :
l'étape a) est réalisée à une température comprise entre 20 °C et 30 °C, à l'abri de la lumière ; à l'étape b), la phase aqueuse est ajoutée en une seule fois à la phase huileuse sous agitation ; à l'étape c), le filtrage est réalisé à l'aide d'un filtre présentant une taille de pore comprise entre 0,20 µm et 0,25 µm ; et l'étape d) de stabilisation est réalisée à une température comprise entre 2 °C et 25 °C.

11. Microémulsion selon les revendications 3, 4 et 6 à 8, destinée à être utilisée comme larmes artificielles.

12. Microémulsion selon les revendications 1 à 4 et 6 à 8, destinée à être utilisée comme véhicule médicamenteux pour une administration oculaire topique.

13. Microémulsion selon l'une quelconque des revendications 5 à 8 destinée à être utilisée dans le traitement de l'hypertension oculaire.

14. Microémulsion selon les revendications 5 à 8 destinée à être utilisée dans le traitement du glaucome.
